# EUROPEAN PATENT APPLICATION

(11) **EP 4 670 774 A1**
(43) Date of publication of application: **31.12.2025**
(21) Application number: 24848220.0
(22) Date of filing: 29.07.2024
(51) Int. Cl.: A61M 60/13

(54) **CATHETER PUMP**

(30) Priority: 01.08.2023 CN 202310957643
(71) Applicant: Magassist Co., Ltd., Suzhou, Jiangsu 215163 (CN)
(72) Inventor: TAN, Renmu, Suzhou, Jiangsu 215163 (CN)
(74) Representative: Grünecker Patent- und Rechtsanwälte PartG mbB
(86) International application number: PCT/CN2024/108118
(87) International publication number: WO 2025/026265

(57) **Abstract**

A catheter pump (1), comprising: a catheter (10); a foldable pump head (30), which comprises a pump housing and a covering film (32) mounted on the pump housing, wherein the pump housing contains an impeller (34) therein and is mounted to a distal end of the catheter (10); and a drive shaft (50), which is rotationally arranged inside the catheter (10) and is connected to the impeller (34). The catheter (10) comprises: a first section (12), which is located inside the covering film (32); a second section (14), which is located near a proximal end of the covering film (32) and is continuous with the first section (12) towards a proximal end of the catheter; and a third section (16), which is continuous with the second section towards the proximal end of the catheter, wherein the stiffness of the first section is less than the stiffness of the second section, and the stiffness of the second section is less than the stiffness of the third section. By means of the provision of the three sections with varying stiffness, the cornering capacity of the catheter during intervention can be improved, and an accumulation caused by deformation of the covering film can also be effectively prevented.

## Description

The present application claims the priority to Chinese Patent Application No. 202310957643.4, titled "CATHETER PUMP", filed on August 1, 2023 with the China National Intellectual Property Administration, which is incorporated herein by reference in its entirety.

### FIELD

Embodiments of the present application relate to the technical field of medical devices, and in particular to a catheter pump for pumping blood.

### BACKGROUND

A catheter pump may be introduced into a patient body through interventional techniques and assist in transporting blood in a circulatory system. Typically, the catheter pump includes a pump head part located inside the patient body and a motor part located outside the patient body. The motor part may be connected to the pump head part via an elongated driving shaft arranged in a catheter, providing power to the pump head part. As an example, when the pump head is deployed in the left ventricle, the catheter pump may pump blood from the left ventricle into the aorta, and when the pump head is deployed in the right ventricle, the catheter pump may pump blood from the inferior vena cava into the pulmonary artery.

The pump head part, the catheter, and the driving shaft are collectively introduced to a predetermined interventional location within the patient body (such as the left ventricle). During the process of introducing these components through blood vessels into the heart, since the blood vessels are not straight, these components need to possess excellent flexibility and elasticity, enabling the interventional components to have good vascular passability.

### SUMMARY

A catheter pump is provided according to an embodiment of the present application, aiming to enhance the performance in one or more aspects of the catheter pump.

A catheter pump is provided according to a first aspect of the present application. The catheter pump includes: a catheter; a collapsible pump head, which includes a pump housing and a film mounted on the pump housing, and the pump housing houses an impeller therein and is mounted to a distal end of the catheter; and a driving shaft, which is rotatably arranged inside the catheter and is connected to the impeller. The catheter includes: a first section, which is located inside the film; a second section, which is located near a proximal end of the film and extends from the first section towards a proximal end of the catheter; and a third section, which extends from the second section towards the proximal end of the catheter. A stiffness of the first section is smaller than that of the second section, and the stiffness of the second section is smaller than that of the third section. Thus, the arrangement of the three sections with different stiffness may improve the ability of the catheter to pass through a bend during the insertion process and effectively prevent accumulation of the film caused by its deformation.

In some embodiments, the first section, the second section, and the third section may be made of the same material.

In some embodiments, an outer diameter of the first section may be smaller than that of the second section, and the outer diameter of the second section may be smaller than that of the third section.

In some embodiments, the second section may have a shape with a dimension gradually increasing from the proximal end towards the distal end.

In some embodiments, the second section may be conical in shape.

In some embodiments, a difference between an average diameter of a proximal end of the second section and an average diameter of a distal end of the second section may be within a range of 0.2mm to 0.4mm, particularly within a range of 0.25mm to 0.35mm.

In some embodiments, a dimension of the second section may be within a range of 5mm to 20mm, particularly within a range of 8mm to 15mm.

In some embodiments, a dimension of the third section may be within a range of 30mm to 80mm, particularly within a range of 50mm to 60mm. In some embodiments, the second section may be located outside the film. In some embodiments, the second section may extend at least partially inside the film.

In some embodiments, the collapsible pump head may be switched between an expanded configuration corresponding to an operating state and a collapsed configuration corresponding to an inserting state. The catheter is pre-bent at the second section and at least partially supports the pump head by a pre-bending stress generated from the pre-bending when the collapsible pump head is in the expanded configuration. Thus, the pump head may be supported by the pre-bending stress provided by the pre-bent portion, improving its centering performance during operation. In some embodiments, an angle at which the catheter may be pre-bent at the second section is configured such that, when the collapsible pump head is in the expanded configuration, the film and the impeller are supported by the second section in a manner that they are substantially perpendicular to an organ tissue supporting the film. This ensures that the pump head is centrally supported and kept away from the inner wall of the heart, preventing the inlet from adhering to the inner wall of the heart or the chordae tendineae of the heart from being drawn into the pump head and becoming entangled.

In some embodiments, the angle at which the catheter may be pre-bent at the second section ranges from 120 degrees to 150 degrees, particularly from 130 degrees to 140 degrees.

In some embodiments, the pre-bending may be formed during a process of integral injection molding of the catheter. This ensures the dimensional accuracy of the pre-bent portion. In some embodiments, in the collapsed configuration, the film and the impeller may be configured to be collapsed in a direction from the distal end of the catheter towards the proximal end of the catheter.

A catheter pump is provided according to a second aspect of the present application. The catheter pump includes: a catheter; a driving shaft rotatably arranged inside the catheter; and a collapsible pump head, including a pump housing and a film mounted to the pump housing. The pump head may be switched between an expanded configuration corresponding to an operating state and a collapsed configuration corresponding to an inserting state, and the film is configured to be collapsed in a direction from a distal end of the catheter towards a proximal end of the catheter when the collapsible pump head is in the collapsed configuration. The catheter includes: a distal-adjacent portion extending from a vicinity of the film to the distal end of the catheter; and a proximal-adjacent portion continuous with the distal-adjacent portion and extending from the vicinity of the film towards the proximal end of the catheter. A stiffness of the distal-adjacent portion is smaller than that of the distal-adjacent portion.

### BRIEF DESCRIPTION OF THE DRAWINGS

The above and other objects, features, and advantages of the embodiments of the present application will become readily comprehensible by reading the following detailed description with reference to the accompanying drawings. In the accompanying drawings, several embodiments of the present application are illustrated in an exemplary manner rather than a restrictive manner.
FIG. 1 is an overall schematic view of a catheter pump according to an embodiment of the present application;
FIG. 2 is a schematic view of a catheter pump according to an embodiment of the present application being deployed in a left ventricle;
FIG. 3 is a partial schematic view of a catheter of a catheter pump according to an embodiment of the present application;
FIG. 4 is a schematic view of a pump head part of a catheter pump according to an embodiment of the present application before being collapsed;
FIG. 5 is a schematic view of a pump head part of a catheter pump according to an embodiment of the present application during a collapsing process.

In the accompanying drawings, identical or corresponding reference numerals denote identical or corresponding parts.

### DETAILED DESCRIPTION OF THE EMBODIMENTS

Preferred embodiments of the present application are described in detail below with reference to the accompanying drawings. Although the preferred embodiments of the present application are shown in the accompanying drawings, it should be understood that the present application may be implemented in various manners and should not be limited to the embodiments set forth herein. In addition, these embodiments are provided to make the present application more thorough and complete and to fully convey the scope of the present application to those skilled in the art.

Unless otherwise defined, all technical and scientific terms used herein have the same meaning as commonly understood by those skilled in the art belonging to the present application. The terms used herein in the specification of the present application are merely for the purpose of describing specific embodiments and are not intended to limit the present application. The term "and/or" used herein includes all arbitrary combinations of one or more relevant items listed.

The term "including" and its variations used herein is inclusive, meaning "including but not limited to". Unless otherwise defined, the term "or" means "and/or". The term "based on" means "at least partially based on". The terms "an exemplary embodiment" and "an embodiment" mean "at least one exemplary embodiment". The term "another embodiment" means "at least one further embodiment".

The terms such as "proximal", "distal", "front", "rear", "inner", and "outer" used in the present application are defined relative to the clinician manipulating the catheter pump. The terms "proximal" and "rear" refer to the part that is relatively closer to the clinician, and the terms "distal" and "front" refer to the part that is relatively farther away from the clinician. For example, the portion of the catheter positioned outside the body is located at a proximal or front end, and the portion of the catheter inserted into the body is located at a distal or rear end. Furthermore, it should be understood that the orientations such as "proximal", "distal", "rear", "front", "inner", and "outer" are defined for convenience of description, and the catheter pump may be used in many orientations and positions. Therefore, these terms for expressing relative positional relationships are not restrictive or absolute. In the present application, if the above definitions are explicitly specified and limited otherwise, they shall follow such explicit specifications and limitations. The catheter pump according to the embodiments of the present application is described below with the accompanying drawings.

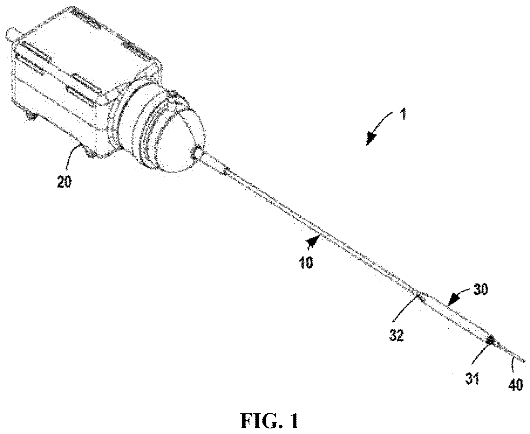

FIG. 1 is an overall schematic view of a catheter pump 1 according to an embodiment of the present application. Generally, the catheter pump 1 needs to be at least partially introduced into a patient body, for example, it may be deployed at a predetermined location in the left or right ventricle to pump blood, thereby at least partially replacing the pumping function of the heart.

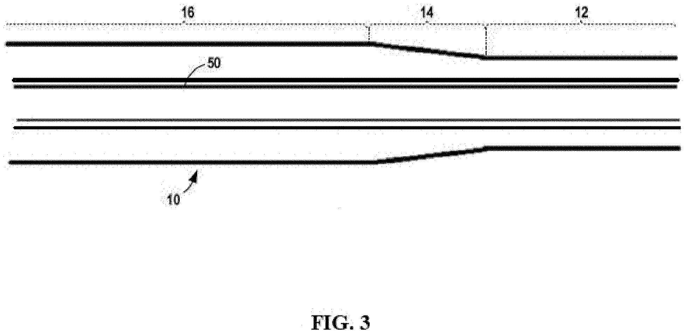

The catheter pump 100 includes a catheter 10, a motor 20, and a pump head 30. The motor 20 may be connected to a proximal end of the catheter 10 via a coupler and is configured as a power component to provide a driving force. A distal end of the catheter 10 is connected to the pump head 30. Inside the catheter 10, a driving shaft 50 (referring to FIG. 3) is provided, with one end of the driving shaft 50 being connected to a power output end of the motor 20. The distal end of the catheter 10 is connected to the pump head 30.

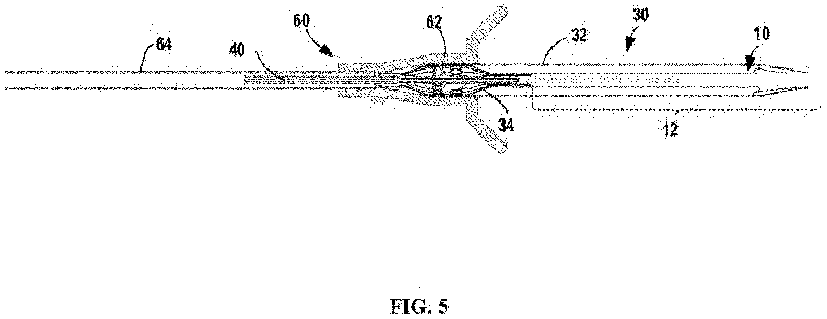

The pump head 30 includes a pump housing and an impeller 34 (referring to FIG. 5) mounted inside the pump housing. The impeller 34 is connected to the other end of the driving shaft 50. Thus, when the driving shaft 50 rotates, it drives the impeller to rotate together. The pump housing includes a metal grid-like framework made of alloys such as nickel, titanium and the like, for example. The metal grid of the pump housing features a mesh design. A film 32 is mounted on the pump housing and, together with the pump housing, defines an inlet 31 for blood intake and an outlet 33 for blood discharge. In some embodiments, the film 32 covers middle and rear portions of the pump housing (i.e., the portion close to a proximal end of the pump housing), such that the outlet 33 for blood is formed at a proximal end of the film 32, and the inlet 31 for blood is formed at a portion, uncovered by the film 32, of a front end (i.e., a distal end) of the pump housing, for example, through the mesh of the pump housing. When the driving shaft 50 drives the impeller to rotate, blood enters a fluid channel defined by the film 32 through the inlet 31 and flows out from the outlet 32 after passing through the film as the impeller rotates.

In some embodiments, the pump head 30 may be collapsible, which offers advantages for the insertion of the catheter pump 1 into the human body. The pump head 30 and a front end portion of the catheter 10 of the catheter pump are introduced into and retained within the patient body, so it is desirable for outer circumferential dimensions of the pump head 30 and the catheter 10 to be as small as possible. The pump head 30 and the catheter 10 of smaller dimensions mean that the pump head 30 and the catheter 10 may be introduced into the patient body through a smaller puncture site, reducing the pain caused to the patient during the introduction process and minimizing complications resulting from an excessively large puncture site. According to the embodiments of the present application, the pump head 30 may have a collapsed configuration and an expanded configuration. In the collapsed configuration, the pump head 30 is in a collapsed state and occupies the smallest possible outer dimension, corresponding to the introduction process of the catheter pump 10. In the expanded configuration, the pump head 30 transitions from the collapsed configuration to an expanded state, corresponding to an operating state of the catheter pump 10, in which the blood pumping channel of the pump head 30 is unobstructed and suitable for pumping blood.

In some embodiments, the pump housing of the pump head 30 may be made of alloy materials such as nickel-titanium. The pump head 30 may be implemented as a multi-mesh design, and may be expanded by virtue of the shape memory properties of the nickel-titanium alloy. Blades of the impeller are made of flexible materials or shape memory materials and may be collapsed relative to a hub. When the pump head 30 is in the collapsed configuration, the blades of the impeller are positioned close to the hub to reduce the occupied dimension. After the external force for constraining the blades of the impeller is removed, the stored energy in the blades is released, causing the blades to expand and recover to the expanded state.

The catheter pump 1 further includes a protective tip 40 connected to a distal end of the pump head 30. During the insertion of the catheter pump 1 into a predetermined location within the human body, the protective tip 40 may guide the insertion of the catheter pump. After the catheter pump 1 is inserted into the predetermined location within the human body (i.e., after the catheter pump is deployed in place), during the operation of the catheter pump 1, the protective tip 40 may maintain the posture of the pump head in the heart, preventing the pump head from adhering to the inner wall of the heart or the chordae tendineae of the heart from being drawn into the pump head, which may cause potential hazards. The protective tip 40 is configured to be soft so as not to harm patient's tissues. In some embodiments, the flexible end of the protective tip 40 is supported on the inner wall of the ventricle in a non-invasive or atraumatic manner, separating the inlet 31 of the pump head 30 from the inner wall of the ventricle. In the illustrated embodiment, the protective tip 40 has a straight shape. It should be understood that the illustrated shape is merely exemplary, and the protective tip 40 may have any other suitable shape.

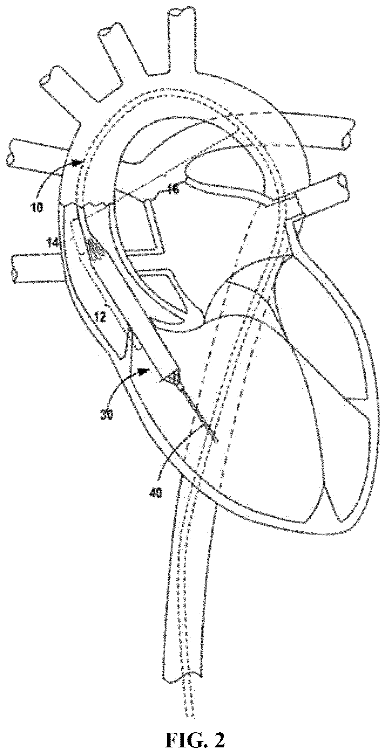

FIG. 2 is a schematic view of the catheter pump 1 according to an embodiment of the present application being deployed in the left ventricle. It should be understood that the illustrated embodiment is merely exemplary, and the catheter pump may also be introduced into other target locations within the patient body as desired, such as the right ventricle, blood vessels, or other organs, through interventional procedures.

Considering that the path from outside the human body to the predetermined location within the human body for the insertion of the catheter pump 1 is not a straight line, the catheter 10 of the catheter pump 1 is generally made of flexible materials to provide sufficient contour-following capability. Through extensive experiments, the inventors of the present application have found that when the catheter 10 has a constant stiffness throughout its length, particularly over a considerable length from the pump head to the proximal end of the catheter, the ability of the catheter 10 to pass through the blood vessel is unsatisfactory, namely its bending capacity is unsatisfactory.

Based on the deployment position requirements of the catheter pump 1, it is desirable for the catheter 10 to have certain stiffness. When the catheter pump 1 is in the operating state shown in FIG. 2, the pump head 30 of the catheter pump 1 should be centered as much as possible relative to the tissue in the heart that supports the pump head 30. One of the purposes of centering is to keep the pump head away from the inner wall of the heart, preventing the inlet from adhering to the inner wall of the heart or the chordae tendineae of the heart from being drawn into the pump head and becoming entangled. However, during the operation of the catheter pump 1, the pumping action of the blood fluid generates a significant reaction force on the pump head 30, causing the posture of the pump head 30 to deflect. When the catheter 10 has sufficient stiffness, the combined effect of the catheter's stiffness and the protective tip 40 can help maintain the posture of the pump head as much as possible.

On the other hand, it is desirable for the catheter 10 to be as flexible as possible to have better contour-following capability. In the embodiment shown in FIG. 2, when deploying the catheter 10 along the contour of the blood vessel wall to the state shown in FIG. 2 through interventional procedures, the catheter 10 needs to pass through regions of the blood vessel with a large curvature, such as the aortic arch region shown in FIG. 2. In these regions with large curvature, there is a high risk that the catheter 10 will come into contact with the inner wall of the aortic arch, preventing further advancement. In such cases, it is desirable for the catheter 10 to have sufficiently low stiffness to exhibit good bending capability.

To address the above issues, a catheter 10 with variable stiffness is provided according to an embodiment of the present application. Specifically, the catheter 10 has a larger stiffness at a proximal-adjacent portion thereof, and the catheter 10 has a smaller stiffness at a distal-adjacent portion thereof than that at the proximal-adjacent portion. Herein, the term "proximal-adjacent portion" may be understood as being defined relative to a region where the film of the pump head 30 is located, referring to a portion that is closer to the proximal end relative to the region occupied by the film of the pump head 30. In some embodiments, as shown in FIG. 2, the "proximal-adjacent portion" may correspond to the area indicated by reference numeral 16 (i.e., a third section). The area indicated by reference numeral 16 may correspond to the region with the greatest curvature during deployment into the heart (i.e., this region has the highest demand for the bending capability of the catheter 10). In the deployment scenario shown in FIG. 2, it may correspond to the region extending from the aortic arch to the vicinity of the pump head. The stiffness of the portion of the catheter 10 extending from the section 16 to the coupling part of the motor 20 may be equal to or larger than that of the section 16. Similarly, the "distal-adjacent portion" may be understood as the portion that is closer to the distal end relative to the area where the film of the pump head 30 is located. In some embodiments, as shown in FIG. 2, the "distal-adjacent portion" may correspond to the regions indicated by reference numerals 12 and 14.

According to the present application, since the "proximal-adjacent portion" occupies a relatively large region of the catheter 10, it may provide sufficient foundational stiffness for the catheter. Moreover, compared to the area adjacent to the pump head, this region with foundational stiffness may provide adequate stiffness support to the pump head 30, ensuring that the pump head remains "centered" during operation of the catheter pump. On the other hand, the "distal-adjacent portion", which corresponds to the area where the pump head is located and/or the area adjacent to the pump head, occupies only a part of the distal region of the catheter 10. Due to the smaller stiffness of the "proximal-adjacent portion", good bending capability is provided at the front end of the catheter. This ensures that the catheter 10 may be guided to the deployment position of the catheter pump 1 without difficulty.

In some embodiments, differences in stiffness may be achieved through different materials. That is, the distal-adjacent portion of the catheter and the proximal-adjacent portion of the catheter may be made of materials with different stiffness, with stiffness variations provided by material differences. Additionally or alternatively, the distal-adjacent portion of the catheter and the proximal-adjacent portion of the catheter may be made of the same material, but with different dimensions to achieve different stiffness. For example, the distal-adjacent portion of the catheter may have an outer diameter larger than that of the proximal-adjacent portion.

In some embodiments, the "distal-adjacent portion" may be further divided into multiple regions with different stiffness to further enhance the performance of the catheter pump. In some embodiments, as shown in FIG. 2, the "distal-adjacent portion" indicated by reference numerals 12 and 14 may include a first section 12 and a second section 14 that is located near the proximal end of the film 32 and extends from the first section 12. The first section 12 is located inside the film 32 and corresponds to the distal end portion of the catheter. The second section 14 is located near the proximal end of the film 32 and extends from the first section 12. Here, the second section 14 corresponds to a transition region between the third section 16 and the first section 12. The first section 12 and the second section 14 may have different stiffness. Specifically, the stiffness of the first section 12 is lower than that of the second section 14, and the stiffness of the second section 14 is lower than that of the third section 16. By providing multiple regions with different stiffness, it is possible to address the issues caused by the collapsing of the collapsed pump head 30 and achieve other additional technical effects, which will be elaborated in detail later.

In some embodiments, differences in stiffness may be achieved through the selection of different materials. That is, the first section 12, the second section 14, and the third section 16 may be made of different materials, with stiffness variations provided by material differences. In other embodiments, the first section 12, the second section 14, and the third section 16 may be made of the same material, but they may be formed with different dimensions to provide different stiffness. For example, the outer diameter of the first section 12 may be smaller than that of the second section 14, and the outer diameter of the second section 14 may be smaller than that of the third section 16.

In some embodiments, the second section 14 has a shape with a dimension gradually increasing from the proximal end towards the distal end. In some embodiments, as shown in FIG. 3, the second section 14 has a conical (including a frustum) shape. This tapered shape is more conducive to eliminating or reducing deformation accumulation of the film. In some embodiments, the difference between the average diameter of the proximal end of the second section 14 and the average diameter of the distal end of the second section 14 is within the range of 0.2mm to 0.4mm, particularly within the range of 0.25mm to 0.35mm. Through extensive testing, it has been found that the above numerical ranges are effective in improving the bending capability of the catheter and reducing the deformation accumulation of the film. It should be understood that the illustrated shapes are merely exemplary, and the first section 12, the second section 14, and the third section 16 may be formed into any appropriate shapes as long as the above stiffness requirements are met. In some embodiments, the second section 14 is located near the proximal end of the film 32. The stiffness difference between the second section 14 and the first section 12 helps compensate for or alleviate the deformation accumulation of the film during collapsing of the pump head 30.

In some embodiments, the third section 16 corresponds to the region of the catheter with high bending requirements. In the deployment scenario shown in FIG. 2, it may correspond to the region that extends from the aortic arch to the vicinity of the pump head. In some embodiments, the dimension of the third section 16 is within the range of 30mm to 80mm, particularly within the range of 50mm to 60mm. Experiments conducted on the above dimension range have shown that the dimension range may meet the stiffness requirements. It should be understood that the dimension range of the third section is merely exemplary and can be set to other appropriate ranges based on the application scenarios of the catheter pump and individual differences. Herein, the dimension refers to the length of each section.

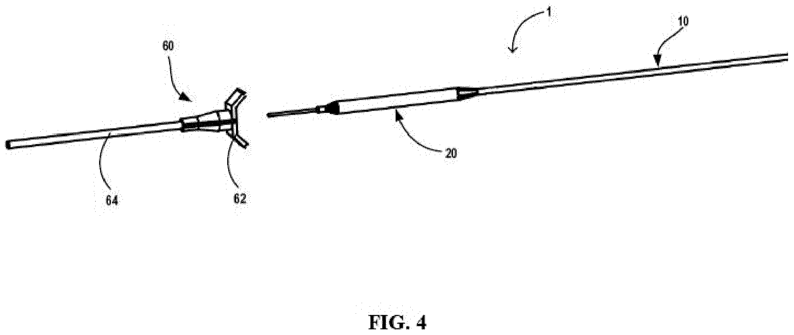

During the process of inserting the pump head 30 of the catheter pump along the blood vessel into the expected deployment position, the impeller and the housing of the pump head 30 are in a collapsed state (for example, the impeller and the housing are collapsed in a direction from the distal end towards the proximal end, as shown in FIGS. 4 and 5, which will be described later), and the film 32 is also collapsed accordingly. However, due to the inherent flexibility of the film 32, when the pump head 30 is advanced along the blood vessel, the film 32 may undergo deformations caused by factors such as blood and/or propulsive force, which is undesirable. On the one hand, these deformations not only increase the resistance to the advancement of the pump head 30. Moreover, in a case of severe deformation, the deformation accumulation may adversely affect the performance of the pump head 30. By providing the second section 14 with a larger stiffness than the first section 12, the above deformation accumulation of the film may be alleviated or eliminated. Furthermore, by providing the second section 14 with a smaller stiffness than the third section 16, the bending capability of the catheter may be improved.

In some embodiments, the second section 14 may be located outside the proximal end of the film 32 and adjacent to the proximal end of the film 32. In some embodiments, the second section 14 extends at least partially inside the film 32. In some embodiments, the dimension of the second section 14 is within the range of 5mm to 20mm, particularly within the range of 8mm to 15mm. Through extensive testing, it has been found that the above-mentioned numerical ranges are effective in improving the bending capability of the catheter and in reducing the deformation accumulation of the film.

In some embodiments, the catheter 10 further adopts some auxiliary measures at the second section 14 to further enhance the performance of the second section 14. In some embodiments, the catheter 10 undergoes a pre-bending at the second section 14. The term "pre-bending" here refers to a forming process through which, when an external force is applied to straighten the catheter 10 (for example, during the process of inserting the catheter 10 into the human body), the catheter 10 will be straightened at the second section 14 under the action of the external force, and when the external force for constraining the catheter 10 is removed (for example, after the pump head of the catheter pump 1 has been deployed at the target deployment position), the catheter 10 retains its pre-bent shape at the second section 14.

Through the pre-bending of the second section 14, when the collapsible pump head 30 is in the expanded configuration, the film 32 may be at least partially supported by the pre-bending stress generated in the second section 14. In this case, the posture of the pump head 30 relative to the organ tissue 70 may be ensured. In the expanded configuration, the film 32 and the impeller 34 are supported by the second section 14 in a manner that they are substantially perpendicular to the vascular tissue supporting the film 32. As a result, the pump head may be kept away from the inner wall of the heart, preventing the inlet from adhering to the inner wall of the heart or the chordae tendineae of the heart from being drawn into the pump head and becoming entangled. In some embodiments, the angle at which the catheter 10 is pre-bent at the second section 14 ranges from 120 degrees to 150 degrees, particularly from 130 degrees to 140 degrees. Based on extensive clinical trials, it has been found that when the pump head is at the operating position, the above-mentioned final bending angle enables the pump head to be centrally positioned.

Pre-bending may be achieved in various ways. In some embodiments, the catheter 10 is integrally formed through injection molding, with the pre-bending being formed during the integral injection molding process of the catheter 10. In some embodiments, after the catheter is integrally molded, heat treatment may be applied to the catheter 10 to bend the catheter 10 into a predetermined shape. For example, the catheter 10 may be annealed to form a curved configuration. Other heat treatments for forming the pre-bending may be considered. For instance, the catheter 10 may be heated on a mandrel used for shaping to form the pre-bending in the catheter 10. Considering the dimensional requirements of multiple sections, forming the pre-bending in the catheter 10 during the integral injection molding process of the catheter 10 has advantages in ensuring dimensional accuracy.

After the catheter 10 is pre-bent at a predetermined location, a straightening force may be applied to the catheter 10 to straighten the pre-bent part of the catheter, and after the straightening force is removed, the catheter 10 returns to its pre-molded curved state in a released condition.

As mentioned above, the pump head 30 of the catheter pump 1 according to the embodiments of the present application is a collapsible pump head. Before the catheter pump 1 is introduced into the human body, the pump head 30 needs to be collapsed to reduce the space occupied by the pump head. FIGS. 4 and 5 are schematic views respectively showing the pump head of the catheter pump 1 according to the embodiments of the present application before and after being collapsed.

FIG. 4 is a schematic view of the pump head 30 of the catheter pump 1 according to the embodiments of the present application before being collapsed. As shown in FIG. 4, an introducer 60 may be provided separately, which may include a tapered funnel-shaped base 62 and an insertion tube 64. The shape of the base 62 is suitable for applying force to the pump head 30 to compress it from the expanded state into the collapsed state. FIG. 5 is a schematic view of the pump head 30 of the catheter pump 1 according to the embodiments of the present application during a collapsing process. As shown in FIG. 5, the pump head of the catheter pump 1 is first partially inserted into the introducer 60. After the protective tip 40 of the catheter pump 1 has passed through the base 62 and entered the insertion tube 64, the pump head 30 begins to enter the base 62. A cavity wall of the base 62 comes into contact with the pump head 30, applying a compressive force to the pump head 30. Under the action of the compressive force, the pump head 30 is collapsed in a direction from the distal end towards the proximal end of the catheter 10. Specifically, the pump housing and the impeller 34 of the pump head are collapsed in a direction from the distal end towards the proximal end of the catheter 10, forming a compact configuration.

The collapsed pump head 30 then enters the insertion tube 64, and maintains the compact configuration under the compressive action of the wall of the insertion tube 64. Subsequently, the catheter pump 1 may be continuously advanced to gradually push the pump head and the catheter 10 of the catheter pump 1 to the deployment location where the catheter pump 1 is to be deployed. During the insertion process, considering that the film 32 of the pump head 30 is made of a flexible material, material accumulation of the film 32 may occur at the distal end of the pump head during the further advancement of the pump head and the catheter 10 of the catheter pump 1, which is undesirable. According to the embodiments of the present application, the catheter 10 forms sections 12 and 14 with different stiffness in the region adjacent to the film 32. Specifically, the second section 14 has a larger stiffness than the first section. With the larger stiffness, material accumulation of the film may be effectively reduced or alleviated while ensuring good bending capability of the catheter 10.

In addition, although the operations are depicted in a specific sequence, it should not be understood that these operations are required to be performed in the specific sequence shown or in a sequential order, nor that all illustrated operations must be performed to achieve the desired results. Under certain circumstances, multitasking and parallel processing may be advantageous. Similarly, although several specific implementation details are included in the above discussion, these should not be interpreted as limiting the scope of the present application. Some features described in the context of individual embodiments may further be implemented in combination in one embodiment. Conversely, various features described in the context of a single embodiment may be implemented in multiple embodiments individually or in any suitable sub-combination.

Although the subject matter is described in language specific to structural features and/or logical actions of methods, it should be understood that the subject matter defined in the claims is not limited to the specific features or actions described above. On the contrary, the specific features and actions described above are merely examples for implementing the claims.

Described above are embodiments of the present application, which are exemplary rather than exhaustive, and are not limited to what is disclosed in the embodiments. Various modifications and changes are apparent for those of ordinary skill in the art, without departing from the scope and spirit of the described embodiments. The choice of terms used herein is intended to best explain the principles, practical applications, or technical improvements in the market of each embodiment, or to enable other ordinary skilled in the art to understand the embodiments disclosed herein.

## Claims

1. A catheter pump, comprising:
a catheter (10);
a collapsible pump head (30), comprising a pump housing and a film (32) mounted on the pump housing, wherein the pump housing accommodates an impeller (34) therein and is mounted to a distal end of the catheter (10); and
a driving shaft (50), rotatably arranged inside the catheter (10) and connected to the impeller (34);
wherein the catheter (10) comprises: a first section (12) located inside the film (32); a second section (14) located near a proximal end of the film (32) and extending from the first section (12) towards a proximal end of the catheter (10); and a third section (16) extending from the second section (14) towards the proximal end of the catheter (10), a stiffness of the first section (12) is smaller than that of the second section (14), and the stiffness of the second section (14) is smaller than that of the third section (16).

2. The catheter pump according to claim 1, wherein the first section (12), the second section (14) and the third section (16) are made of a same material.

3. The catheter pump according to claim 2, wherein an outer diameter of the first section (12) is smaller than that of the second section (14), and the outer diameter of the second section (14) is smaller than that of the third section (16).

4. The catheter pump according to claim 3, wherein the second section (14) has a shape with a dimension gradually increasing from the proximal end towards the distal end.

5. The catheter pump according to claim 4, wherein the second section (14) has a conical shape.

6. The catheter pump according to claim 4, wherein a difference between an average diameter of a proximal end of the second section (14) and an average diameter of a distal end of the second section (14) ranges from 0.2mm to 0.4mm, particularly from 0.25mm to 0.35mm.

7. The catheter pump according to claim 1, wherein a dimension of the second section (14) ranges from 5mm to 20mm, particularly from 8mm to 15mm.

8. The catheter pump according to any one of claims 1 to 7, wherein a dimension of the third section (16) ranges from 30mm to 80mm, particularly from 50mm to 60mm.

9. The catheter pump according to any one of claims 1 to 7, wherein the second section (14) is located outside the film (32).

10. The catheter pump according to any one of claims 1 to 7, wherein the second section (14) extends at least partially inside the film (32).

11. The catheter pump according to any one of claims 1 to 7, wherein the collapsible pump head (30) is switchable between an expanded configuration corresponding to an operating state and a collapsed configuration corresponding to an inserting state, the catheter (10) is pre-bent at the second section (14) and at least partially supports the pump head (30) by a pre-bending stress generated from the pre-bending when the collapsible pump head (30) is in the expanded configuration.

12. The catheter pump according to claim 11, wherein an angle at which the catheter (10) is pre-bent at the second section (14) is configured such that, when the collapsible pump head is in the expanded configuration, the film (32) and the impeller (34) are supported by the second section (14) in a manner that they are substantially perpendicular to an organ tissue supporting the film (32).

13. The catheter pump according to claim 11, wherein the angle at which the catheter (10) is pre-bent at the second section (14) ranges from 120 degrees to 150 degrees, particularly from 130 degrees to 140 degrees.

14. The catheter pump according to claim 11, wherein the pre-bending is formed during a process of integral injection molding of the catheter (10).

15. The catheter pump according to claim 11, wherein in the collapsed configuration, the film (32) and the impeller (34) are configured to be collapsed in a direction from the distal end of the catheter (10) towards the proximal end of the catheter (10).

16. A catheter pump, comprising:
a catheter (10);
a driving shaft (50), rotatably arranged inside the catheter (10); and
a collapsible pump head (30), comprising a pump housing and a film (32) mounted to the pump housing, wherein the pump head (30) is switchable between an expanded configuration corresponding to an operating state and a collapsed configuration corresponding to an inserting state, and the film (32) is configured to be collapsed in a direction from a distal end of the catheter (10) towards a proximal end of the catheter (10) when the collapsible pump head (30) is in the collapsed configuration;
wherein the catheter (10) comprises: a distal-adjacent portion extending from a vicinity of the film (32) to the distal end of the catheter; and a proximal-adjacent portion continuous with the distal-adjacent portion and extending from the vicinity of the film (32) towards the proximal end of the catheter (10), with a stiffness of the distal-adjacent portion being smaller than that of the distal-adjacent portion.
